(19) Europäisches Patentamt
European Patent Office
Office européen des brevets

(11) Veröffentlichungsnummer: **0 173 871**
**B1**

(12) # EUROPÄISCHE PATENTSCHRIFT

(45) Veröffentlichungstag der Patentschrift: **17.11.88**

(51) Int. Cl.[4]: **C 07 D 277/80**

(21) Anmeldenummer: **85109812.9**

(22) Anmeldetag: **05.08.85**

(54) **Verfahren zur Herstellung von Benzothiazolsulfenamiden.**

(30) Priorität: **18.08.84 DE 3430435**

(43) Veröffentlichungstag der Anmeldung:
**12.03.86 Patentblatt 86/11**

(45) Bekanntmachung des Hinweises auf die Patenterteilung:
**17.11.88 Patentblatt 88/46**

(84) Benannte Vertragsstaaten:
**BE DE FR GB IT NL**

(56) Entgegenhaltungen:
**EP - A - 0 004 896**
**EP - A - 0 131 776**
**DE - A - 2 356 686**
**DE - A - 2 744 423**
**DE - B - 1 695 533**

**CHEMICAL ABSTRACTS, Band 92, Nr. 9, 3. März 1980, Seite 667, Zusammenfassung Nr. 76380u, Columbus, Ohio, US; V.A. IGNATOV et al.: "Study of the reaction by bis(2-benzothiazolyl)disulfide with amines", & IZV. VYSSH. UCHEBN. ZAVED., KHIM. KHIM. TEKHNOL. 1979, 22(9), 1067-1070 (Kat. D,A)**

(73) Patentinhaber: **BAYER AG, Konzernverwaltung RP Patentabteilung, D-5090 Leverkusen 1 Bayerwerk (DE)**

(72) Erfinder: **Wüst, Alfredo, Dr., Siebengebirgsweg 14, D-5064 Rosrath (DE)**
Erfinder: **van Osselaer, Tony, Dr., Tuinlaan 62, B-9180 Belsele (BE)**

**EP 0 173 871 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein Verfahren zur Herstellung von Benzothialzolsulfenamiden aus Dibenzothiazolyldisulfiden und wasserlöslichen Aminen.

Benzothiazolsulfenamide werden üblicherweise durch oxidative Kupplung von 2-Mercaptobenzothiazolen, deren Alkalisalzen oder Disulfiden und primären oder sekundären Aminen hergestellt (DE-A-2 356 686 und DE-A-2 744 423). Dabei werden entweder nur mässige Ausbeuten in Bezug auf die Mercaptobenzthiazol-Basis erzielt und die Produkte sind durch Nebenprodukte verunreinigt, die die Lagerstabilität des Sulfenamids beeinträchtigen oder es müssen aufwendige und unökonomische Verfahren mit komplizierten Reinigungsschritten angewendet werden

Auf das Amin bezogen werden nach den bekannten Verfahren Ausbeuten von höchstens 90% erreicht. Dies ist ein wesentlicher Nachteil, da für die bekannten Sulfenamide, die bekanntlich als Vulkanisationsbeschleuniger eingesetzt werden, Amine Verwendung finden, deren Herstellung mindestens ebenso kostspielig ist wie die Herstellung des Mercaptobenzthiazolteils.

Es ist weiterhin aus Izv. vyssh. Uchebn. Zaved., Khimiya i khim. tekhnol., 22 (1979), 9, 1067–1070 bekannt, Dibenzothiazolyldisulfid (MBTS) mit Aminen in An- und Abwesenheit von Alkali und in Abwesenheit eines Oxidationsmittels umzusetzen.

Ohne Alkali wurde mit Morpholin als Amin eine Ausbeute von 86% bezogen auf den Umsatz erzielt, der allerdings nur 50% beträgt, denn je Mol erzeugtes Sulfenamid entsteht ein Mol Morpholinsalz der Mercaptobenzthiazols, das noch mit Natronlauge in einem separaten Arbeitsgang aufgearbeitet werden muss.

Setzt man Natronlauge zu, um das Natriumsalz des Mercaptobenzthiazols (NaMBT) als wiederverwendbares Nebenprodukt zu erzeugen und die einzusetzende Aminmenge zu reduzieren, so sinkt die Ausbeute an Sulfenamid in Abhängigkeit von der Natronlaugemenge bis auf 10%.

Aufgabe der Erfindung ist es, ein ökonomisches Verfahren zur Herstellung von reineren Benzothiazolylsulfenamiden in verbesserter Ausbeute und mit verbesserter Lagerstabilität bereitzustellen.

Überraschenderweise wurde nun gefunden, dass die Umsetzung von MBTS mit wasserlöslichen Aminen in Abwesenheit von Oxidationsmitteln und in Gegenwart von wässrigem Alkali in hoher Ausbeute zu reinen und stabilen Benzthiazolsulfenamiden dann gelingt, wenn bei der Reaktionsführung bestimmte Bedingungen eingehalten werden.

Gegenstand der Erfindung ist daher ein Verfahren zur Herstellung von Benzthiazolsulfenamiden der allgemeinen Formel

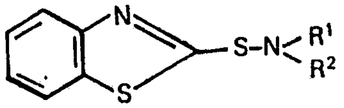

worin $R^1$ = H und $R^2$ = $C_1$–$C_5$ Alkyl, Cyclopentyl, Cyclohexyl; $R^1 = R^2 = CH_3$ oder $C_2H_5$;

oder $R^1$ und $R^2$ zusammen mit dem N-Atom einen Piperazino-, Morpholino-, Pyrrolidino- oder Piperidinorest bedeuten, aus Dibenzothiazolyldisulfid und einem wasserlöslichen Amin in Abwesenheit eines Oxidationsmittels und in Anwesenheit von wässrigem Alkali bei einer Temperatur von 0 bis 80 °C, dadurch gekennzeichnet, dass man das Amin oder die Lösung des Amins in Wasser vorlegt und gleichzeitig Dibenzothiazolyldisulfid und wässriges Alkali aus getrennten Vorratsgefässen so zugibt, dass sich ein pH-Wert von 10 bis 13 einstellt, und als Amin Methylamin, Ethylamin, Dimethylamin, Diethylamin, n-Propylamin, Isopropylamin, Butylamine, Pentylamine, Cyclopentylamine, Cyclohexylamin, Piperazin, Morpholin, Pyrrolidin oder Piperidin verwendet.

Das Verhältnis Wasser zu Amin beträgt vorzugsweise höchstens 20:1, insbesondere höchstens 10:1 Gew.-Teile.

Das wässrige Alkali wird so zugegeben, dass sich ein pH-Wert von vorzugsweise 11 bis 12 einstellt. Die Reaktion wird bei einer Temperatur von vorzugsweise 20 bis 60 °C durchgeführt.

Das entstehende Sulfenamid wird abgetrennt; aus der Mutterlauge werden überschüssiges Amin (durch Destillation) und Mercaptobenzthiazol (durch Fällung mit Säure) zurückgewonnen.

Als wässriges Alkali kommen insbesondere Natronlauge und Kalilauge in Frage.

Als Amine kommen in Wasser mindestens zu 1 Gew.-% lösliche Amine mit einem $pK_s < 7$ (in wässriger Lösung) infrage, nämlich Methylamin, Ethylamin, Dimethylamin, Diethylamin, n-Propylamin, Isopropylamin, Butylamine, Pentylamine, Cyclopentylamin, Cyclohexylamin, Piperazin, Morpholin, Pyrrolidin und Piperidin.

Das erfindungsgemässe Verfahren ist insbesondere bei Verwendung von Cyclohexylamin, tert.-Butylamin, Isopropylamin und Morpholin wertvoll.

Beispiel 1

N-Cyclohexyl-2-benzothiazolsulfenamid

In einem 2 l-Mehrhalskolben mit Rührer, Tropftrichter, Kühler, Thermometer und pH-Elektrode wurden 100 g Cyclohexylamin und 300 g Wasser vorgelegt und auf 50 °C erhitzt.166 g Dibenzothiazolyldisulfid wurden in kleinen Portionen innerhalb von 10 bis 15 Minuten eingetragen. Parallel dazu tropfte man 40 g 50 gew.-%ig Natronlauge zu, so dass der pH zwischen 11 und 12 lag. Nach beendeter Zugabe rührte man bis zu 2 Stunden nach, saugte den Niederschlag ab, wusch mit Wasser nach und trocknete im Vakuumtrockenschrank bei 60 °C. Aus Mutterlauge und Waschwasser wurde das Cyclohexylamin durch Destillation zurückgewonnen. Aus dem Destillationssumpf wurde das Mercaptobenzothiazol durch Zugabe von 25%iger Schwefelsäure gefällt, abgesaugt, mit Wasser gewaschen und im Vakuumtrockenschrank bei 60 °C getrocknet.

Ausbeute 96 bis 97% der Theorie, Gehalt 97 bis 98%, Amingehalt 0,2%, Unlösliches in Ethanol 0%.

Cyclohexylamin-Rückgewinnung: 97%, Mercaptobenzolthiazol-Rückgewinnung 100%.

Nach 24-stündiger Alterung bei 60 °C in wasserfeuchter Atmosphäre: Gehalt 96,7%, Amingehalt 0,4%, Unlösliches in Ethanol 0%.

Beispiel 2

N-tert.-Butyl-2-benzothiazolsulfenamid

In einer Apparatur nach Beispiel 1 wurden 219 g t-Butylamin vorgelegt. Bei Raumtemperatur (20 bis 30 °C) wurden 166 g Dibenzothiazolyldisulfid in kleinen Portionen innerhalb von 10 bis 15 Minuten unter Rühren eingetragen. Parallel hierzu wurden insgesamt 240 g 8,3 gew.-%iger Natronlauge zugegeben, so dass ein pH-Wert von mindestens 11 eingehalten wurde. Man liess 2 Stunden nachrühren, saugte den Niederschlag ab, wusch mit Wasser nach und trocknete im Vakuumtrockenschrank bei 60 °C.

Ausbeute 96% bezogen auf Dibenzothiazolyldisulfid, 95,5% bezogen auf t-Butylamin. Gehalt 98,4%, Amingehalt 0,3%, Unlösliches in Ethanol 0%, Schmelzpunkt 109 bis 111 °C.

Nach 24-stündiger Alterung bei 60 °C in wasserfeuchter Atmosphäre: Gehalt 98,2%, Amingehalt 0,3%, Unlösliches in Ethanol 0%.

Beispiel 3

4-(2-Benzothiazolylthio)-morpholin

In einer Apparatur nach Beispiel 1 wurden 261 g Morpholin vor gelegt. Bei Raumtemperatur wurden innerhalb von 20 Minuten unter Rühren 166 g Dibenzothiazolyldisulfid und parallel dazu 240 g 8,3 gew.-%ige Natronlauge zugegeben.

Nach zweistündigem Nachrühren wurde der Feststoff abgesaugt, mit Wasser gewaschen und im Vakuumschrank bei 50 °C getrocknet.

Ausbeute 96,5% bezogen auf Dibenzothiazolyldisulfid, 94% bezogen auf Morpholin. Gehalt 98%, Amingehalt 0,2%, Unlösliches in Ethanol 0%, Schmelzpunkt 84 bis 86 °C.

Beispiel 4

N-Isopropyl-2-benzothiazolsulfenamid

In eine Apparatur nach Beispiel 1 wurden 236 g Isopropylamin und 100 g Wasser vorgelegt. Bei 20 bis 30 °C wurden innerhalb von 20 Minuten unter Rühren 166 g Dibenzolthiazolyldisulfid und parallel dazu 80 g 25 gew.-%ige Natronlauge zugegeben. Nach beendeter Umsetzung liess man 2 Stunden nachrühren und saugte den Niederschlag ab, der anschliessend mit Wasser gewaschen und im Vakuumtrockenschrank bei 60 °C getrocknet wurde.

Ausbeute 97,5% bezogen auf Dibenzothiazolyldisulfid, 95% bezogen auf Isopropylamin. Gehalt 97,7%, Amingehalt 0,2%, Unlösliches in Ethanol 0%, Schmelzpunkt 94 bis 96 °C.

Nach Alterung (s. Beispiel 1) Gehalt 96,2%, Amingehalt 0,3%, Schmelzpunkt 93 bis 96 °C.

Beispiel 5

N-tert.-Butyl-2-benzothiazolsulfenamid

In einer Apparatur nach Beispiel 1, zusätzlich mit einer Eintragschnecke und einem Überlauf ausgerüstet, wurden 219 g t-Butylamin vorgelegt. Bei Raumtemperatur (20 bis 30 °C) wurden 166 g Dibenzothiazolyldisulfid in kleinen Portionen innerhalb von 10 bis 15 Minuten unter Rühren eingetragen. Parallel hierzu wurden insgesamt 240 g 8,3 gew.-%ige Natronlauge zugegeben, so dass ein pH-Wert von mindestens 11 eingehalten wurde. Dann führte man stündlich 332 g Dibenzolthiazolyldisulfid, 438 g t-Butylamin und 480 g 8,3 gew.-%ige Natronlauge unter Rühren bei 20 bis 30 °C zu. Das Reaktionsgemisch wurde kontinuierlich über den Überlauf abgenommen und durch Filtration in Feststoff und Mutterlauge getrennt. Nach Waschen und Trocknen wie in Beispiel 2 erhält man N-tert.-Butyl-benzothiazolsulfenamid.

Gehalt 98,5%, Amingehalt 0,15%, Unlösliches in Ethanol 0%, Schmelzpunkt 107 bis 110 °C, nach Alterung Gehalt 98,1%, Amingehalt 0,16%, Unlösliches in Ethanol 0%.

**Patentansprüche**

1. Verfahren zur Herstellung von Benzthiazolsulfenamiden der allgemeinen Formel

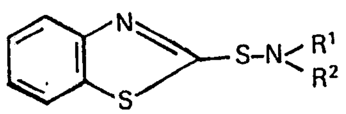

worin $R^1$ = H und $R^2$ = $C_1$–$C_5$ Alkyl, Cyclopentyl, Cyclohexyl; $R^1 = R^2 = CH_3$ oder $C_2H_5$;

oder $R^1$ und $R^2$ zusammen mit dem N-Atom einen Piperazino-, Morpholino-, Pyrrolidino- oder Piperidinorest bedeuten, aus Dibenzothiazolyldisulfid und einem wasserlöslichen Amin in Abwesenheit eines Oxidationsmittels und in Abwesenheit von wässrigem Alkali bei einer Temperatur von 0 bis 80 °C, dadurch gekennzeichnet, dass man das Amin oder die Lösung des Amins in Wasser vorlegt und gleichzeitig Dibenzothiazolyldisulfid und wässriges Alkali aus getrennten Vorratsgefässen so zugibt, dass sich ein pH-Wert von 10 bis 13 einstellt, und als Amin Methylamin, Ethylamin, Dimethylamin, Diethylamin, n-Propylamin, Isopropylamin, Butylamine, Pentylamine, Cyclopentylamin, Cyclohexylamin, Piperazin, Morpholin, Pyrrolidin oder Piperidin verwendet.

2. Verfahren nach Anspruch 1, dadurch gekennzeichnet, dass man das wässrige Alkali so zugibt, dass sich ein pH-Wert von 11 bis 12 einstellt, und die Reaktion bei 20 bis 60 °C durchgeführt wird.

3. Verfahren nach Anspruch 1, dadurch gekennzeichnet, dass man als Amin tert.-Butylamin, Cyclohexylamin, Isopropylamin oder Morpholin einsetzt.

## Revendications

1. Procédé de préparation de benzothiazole-sulfénamides de formule générale

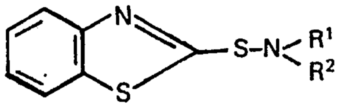

dans laquelle $R^1$ = H et $R^2$ = alkyle en $C_1$– $C_5$, cyclopentyle, cyclohexyle; $R^1$ = $R^2$ = $CH_3$ ou $C_2H_5$;
ou bien $R^1$ et $R^2$ forment ensemble et avec l'atome d'azote un groupe pipérazino, morpholino, pyrrolidino ou pipéridino, à partir du disulfure de dibenzothiazolyle et d'une amine soluble dans l'eau en l'absence d'agent oxydant et en présence d'alcali aqueux à une température de 0 à 80 °C, caractérisé en ce que l'on introduit l'amine ou la solution de l'amine dans l'eau et on ajoute simultanément, à partir de récipients de réserve séparés, le disulfure de dibenzothiazolyle et un alcali aqueux de manière à régler à un pH de 10 à 13, et en ce que l'on utilise en tant qu'amine la méthylamine, l'éthylamine, la diméthylamine, la diéthylamine, la n-propylamine, l'isopropylamine, une butylamine, une pentylamine, la cyclopentylamine, la cyclohexylamine, la pipérazine, la morpholine, la pyrrolidine ou la pipéridine.

2. Procédé selon la revendication 1, caractérisé en ce que l'on ajoute l'alcali aqueux de manière à régler à un pH de 11 à 12 et on effectue la réaction à des températures de 20 à 60 °C.

3. Procédé selon la revendication 1, caractérisé en ce que l'on utilise en tant qu'amine la tert-butylamine, la cyclohexylamine, l'isopropylamine ou la morpholine.

## Claims

1. A process for the production of benzthiazole sulfenamides corresponding to the following general formula

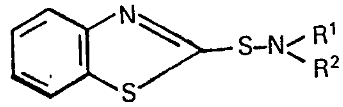

in which $R^1$ = H and $R^2$ = $C_1$–$C_5$ alkyl, cyclopentyl, cyclohexyl; $R^1$ = $R^2$ = $CH_3$ or $C_2H_5$;
or $R^1$ and $R^2$, together with the N-atom, represent a piperazino, morpholino, pyrrolidino or piperidino ring, from dibenzothiazolyl disulfide and a water-soluble amine in the absence of an oxidizing agent and in the presence of aqueous alkali at a temperature of 0 to 80 °C, characterised in that the amine or an aqueous solution of the amine is initially introduced and the dibenzothiazolyl disulfide and aqueous alkali are simultaneously added from separate storage vessels in such quantities that a pH value of from 10 to 13 is established and in that methylamine, ethylamine, dimethylamine, diethylamine, n-porpylamine, isopropylamine, butylamines, pentylamines, cyclopentylamine, cyclohexylamine, piperazine, morpholine, pyrrolidine or piperidine are used as the amine.

2. A process as claimed in claim 1, characterised in that the aqueous alkali is added in such a quantity that a pH value of 11 to 12 is established and in that the reaction is carried out at 20 to 60 °C.

3. A process as claimed in claim 1, characterized in that tert.-butylamine, cyclohexylamine, isopropylamine or morpholine is used as the amine.